# EUROPEAN PATENT APPLICATION

(11) **EP 1 897 611 A1**
(43) Date of publication of application: **12.03.2008**
(21) Application number: 06729882.8
(22) Date of filing: 24.03.2006
(51) Int. Cl.: B01J 13/04, A61K 9/50

(54) **PROCESS FOR PRODUCING MICROCAPSULE, MICROCAPSULE PRODUCTION APPARATUS AND MICROCAPSULE SHEET**

(30) Priority: 14.04.2005 JP 2005116454
(71) Applicant: TORAY ENGINEERING CO., LTD., Chuo-ku Tokyo 103-0021 (JP); Iwade, Takashi, Otsu-shi, Shiga 520-2141 (JP)
(72) Inventor: IZUMIDA, Shinya C/O TORAY ENGINEERING CO.,, Otsu-shi, Shiga; 5202141 (JP)
(74) Representative: Winkler, Andreas Fritz Ernst
(86) International application number: PCT/JP2006/305934
(87) International publication number: WO 2006/112235

(57) **Abstract**

Problems

To accomplish fine treating of microcapsules per se.

Means for Solving Problems

There is provided an apparatus comprising XY-stage adapted to support base material (1), such as paper, and conduct two-dimensional moving thereof; nozzle unit (3) having three liquid droplet feeding nozzles adapted to separately feed liquid droplets containing a first shell material, liquid droplets containing a core material and liquid droplets containing a second shell material from above the base material (1) so as to cause them to spot to the surface of the base material (1); and drying unit (4) adapted to dry the spotted liquid droplets to thereby form first and second shell films.

## Description

### Technical Field

The present invention relates to a process for producing microcapsule, and microcapsule production apparatus, the microcapsule being configured to seal a core material within a fine shell, and to a microcapsule sheet which is configured to arrange microcapsules in a predetermined pattern on a base material.

### Background Art

From the past, microcapsules have become to be used in various fields in view of functions such as preventing degradation of active element in a microcapsule, improving workability, discharging core material when it is required, and gradually discharging core material (discharging core material little by little).

And, a phase separation method, submerged hardening coating method, In Situ polymerization method, interfacial polymerization method and the like are known as a process for producing microcapsule, as is recited in the patent document 1.
Patent Document 1: Japanese Patent Laid-Open No.2003-344881 gazette

### Disclosure of the Invention

### Problems to be Solved by the Invention

The phase separation method is a method for separating polymer-rich phase around core material, the core material consisting of colored charged particles separated within polymer solution and dispersion medium. Thus, the method has a disadvantage that fine treating cannot be applied to the microcapsules per se.

The submerged hardening coating method is a method for hardening polymer by applying polymer hardening test drug and the like around core material within polymer solution. Thus, the method has a disadvantage that fine treating cannot be applied to the microcapsules per se.

The In Situ polymerization method is a method for supplying monomer or polymerization catalyst from one of internal phase and external phase of emulsion in which core material is dispersed and for covering core material with polymer. Thus, the method has a disadvantage that fine treating cannot be applied to the microcapsules per se.

The interfacial polymerization method is a method for supplying monomer from both of internal phase and external phase of emulsion in which core material is dispersed. Thus, the method has a disadvantage that fine treating cannot be applied to the microcapsules per se.

The present invention was made in view of the above problems.

It is a first object of the present invention to provide a process for producing microcapsule and a microcapsule production apparatus which can apply fine treating to the microcapsules per se.

It is a second object of the present invention to provide microcapsule sheet employing microcapsules produced by the process for producing microcapsule or the microcapsule production apparatus.

### Means for solving the Problems

A process for producing microcapsule according to the present invention comprises the steps of forming a first shell film by spotting a liquid droplet containing a first shell material to a base material, forming a core layer by spotting a liquid droplet containing a core material to the first shell film, and forming a second shell film by spotting a liquid droplet containing a second shell material to the base material so that the core layer is contained between the first shell film and the second shell film. Wherein the core layer thus formed may be of one layer, two layers or more layers.

In this case, arbitrary material can be employed as the first shell material and the second shell material. Therefore, fine treating can be applied to the microcapsules per se.

A microcapsule production apparatus according to the present invention comprises first shell film forming means for forming a first shell film by spotting a liquid droplet containing a first shell material to a base material, core layer forming means for forming a core layer by spotting a liquid droplet containing a core material to the first shell film, and second shell film forming means for forming a second shell film by spotting a liquid droplet containing a second shell material to the base material so that the core layer is contained between the first shell film and the second shell film.

Therefore, the microcapsule is formed by forming the first shell film by the first shell film forming means, the core layer by the core layer forming means, and the second shell film by the second shell film forming means. Wherein the core layer thus formed may be of one layer, two layers or more layers.

In this case, arbitrary material can be employed as the first shell material and the second shell material. Therefore, fine treating can be applied to microcapsules per se.

A microcapsule sheet according to the present invention comprises a base material, and microcapsules arranged at a predetermined pattern within a predetermined extent of the base material, the microcapsule being formed by surrounding a core layer with a first shell film and a second shell film.

In this case, microcapsules are arranged only within a required extent of the base material, thus waste in microcapsules is prevented from occurrence.

### Effect of the Invention

The process for producing microcapsule according to the present invention has the characteristic effect such that fine treating can be applied to microcapsules per se.

The microcapsule production apparatus according to the present invention has the characteristic effect such that fine treating can be applied to microcapsules per se.

The microcapsule sheet according to the present invention has the characteristic effect such that waste in microcapsules is prevented from occurrence.

### Best Mode for Carrying Out the Invention

Hereinafter, referring to the attached drawings, we explain embodiments of a process for producing microcapsule, a microcapsule production apparatus, and a microcapsule sheet according to the present invention, in detail.

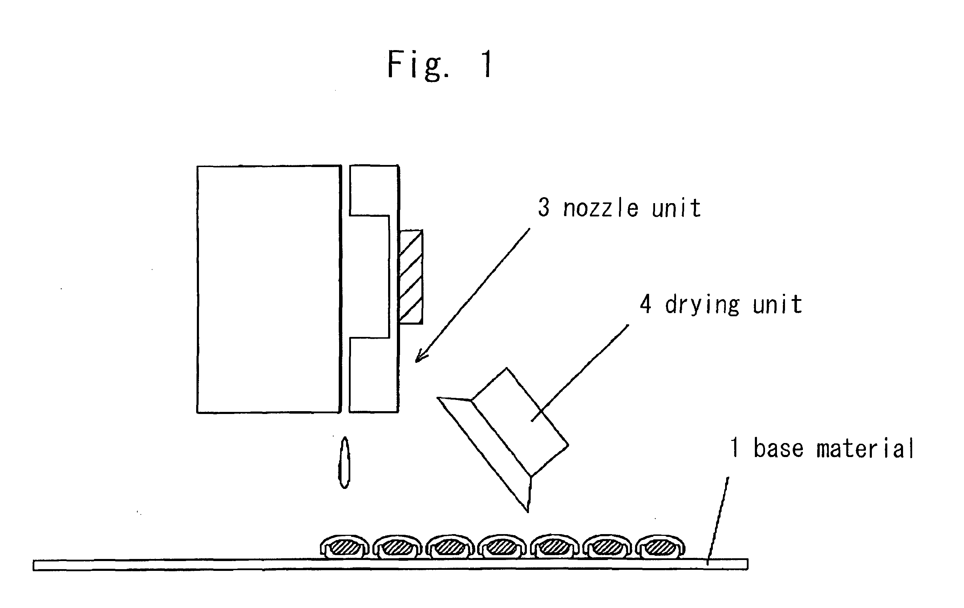

Fig. 1 is a schematic view of an embodiment of a microcapsule production apparatus according to the present invention.

The microcapsule production apparatus comprises an XY-stage (not illustrated) adapted to support a base material 1, such as paper, and conduct two dimensional moving thereof, a nozzle unit 3 having three liquid droplet feeding nozzles adapted to separately feed liquid droplets containing a first shell material, liquid droplets containing a core material, liquid droplets containing a second shell material, from above the base material 1, the liquid droplets containing a first shell material and the liquid droplets containing a second shell material being spotted to the top face of the base material 1, and a drying unit 4 for drying spotted liquid droplets containing a first shell material to form a first shell film, and for drying spotted liquid droplets containing a second shell material to form a second shell film.

The liquid droplet feeding nozzle increases and decreases a volume of a liquid droplet accumulation section using a piezo element or the like, for example, so as to suck liquid from a liquid droplet tank and to discharge the sucked liquid as a liquid droplet, the operation is similar to an ink nozzle employed in an ink jet printer. The liquid droplet feeding nozzle sucks liquid and discharges the sucked liquid as a liquid droplet, thus discharge amount can be controlled with high accuracy.

The nozzle unit 3 has the three liquid droplet feeding nozzles, thus the nozzle unit 3 is configured to move to and fro in correspondence with the arrangement of the three liquid droplet feeding nozzles so that three liquid droplet feeding nozzles are sequentially operated at the same position.

As the drying unit 4, an infrared heater can be employed, for example.

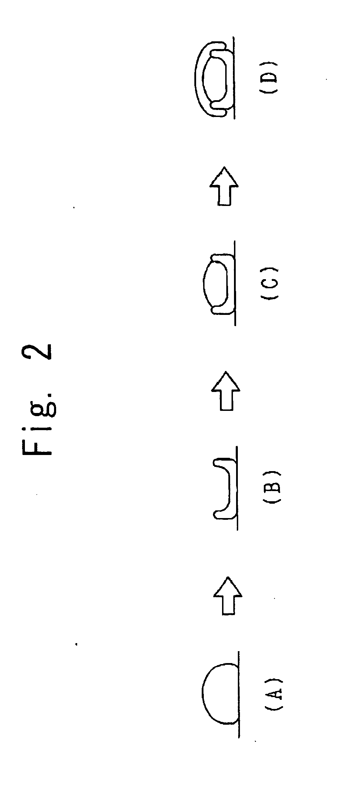

The operation of the microcapsule production apparatus illustrated in Fig. 1 is described with reference to Fig. 2.

The base material 1 is moved to a predetermined position by the XY-stage. After that, liquid droplets including a first shell material is spotted to the base material 1 by driving one of the three liquid droplet feeding nozzles of the nozzle unit 3. Under this condition, the liquid droplet has almost hemisphere shape (refer to (A) in Fig. 2).

After that, the drying unit 4 is operated, thus the surface of the liquid droplet is dried at first so as to form a film. By continuing drying operation thereafter, interior solvent medium and the like of the liquid droplet are evaporated, thus the firstly formed film is made to have concave shape (refer to (B) in Fig. 2). The film may be made to have flat shape depending upon the species of the shell material and the liquid droplet, and the like.

After that, the liquid droplet including a core material is spotted on the first shell film (refer to (C) in Fig. 2) by driving another of the three liquid droplet feeding nozzles of the nozzle unit 3.

After that, the liquid droplet including a second shell material is spotted to the base material 1 and the core layer to cover the core layer by driving further one of the three liquid droplet feeding nozzles of the nozzle unit 3.

After that, by operating the drying unit 4, the liquid droplet including a second shell material is dried, thus the second shell film is formed (refer to (D) in Fig. 2).

A microcapsule is produced on the base material 1 by the above operations.

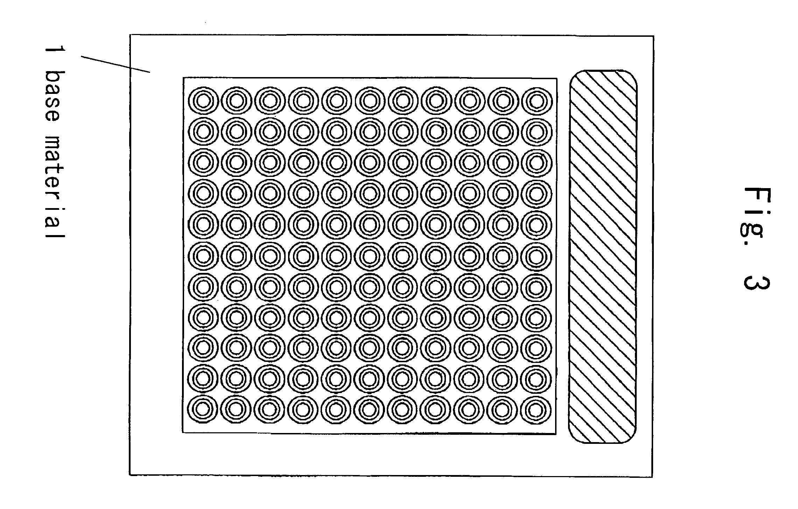

Because the base material 1 can be moved by the XY-stage, only within a predetermined extent on the base material 1, microcapsules are arranged at a predetermined pattern (refer to Fig. 3).

As the use application of the microcapsule thus produced, carbonless paper (carbonless copying paper), pressure measurement film, recording material such as toner, adhesive material such as seal and lock material, insulating material for metal particle, thermal expansion material, heat medium, industrial material such as dimmer glass, thermochromic (temperature-sensitive liquid crystal, temperature-sensitive dye compound), indicator material such as magnetic migration, agricultural chemicals, artificial diet (vitamin class), agroforestry and marin product such as artificial seed, air freshener, cold cream, cosmetic such as lip stick, beauty product, and vitamin class, activated charcoal, artificial body part (enzyme), medical drug such as DDS (drug delivery system), medical products are exemplified.

Depending upon the use application, accordingly selecting the shell material and core material becomes necessary. Especially, for a microcapsule produced by the process for producing microcapsule, or microcapsule production apparatus according to the present invention, the first shell material and the second shell material can be selected independently from one another. Therefore, treating such as material having translucency is employed as one shell material and material having no translucency is employed as the other shell material, for example, depending upon the use application, becomes possible.

As the shell material, various material such as natural polymer such as gelatin, gum Arabic, soda alginate, semisynthetic polymer such as carboxymethyl cellulose, ethyl cellulose, synthetic polymer such as polyvinyl alcohol nylon, polyurethane, polyester, epoxy, melamine-formalin, fat class such as wax, inorganic material such as colloidal silica, can be employed in simple body or in complex depending upon the use application. Of course, it is possible that the shell material is colored by mixing dye or pigment or the like in the shell material.

As the base material 1, soluble and edible material can be employed for medical drug application, for example. Further, it is possible that microcapsules are formed into sheet shape by transferring microcapsules arranged on a film serving as the base material 1 to another base material. Furthermore, it is possible that microcapsules are formed into sheet shape by capturing microcapsules with another binder, the microcapsules being arranged on a film serving as the base material 1, then microcapsules are provided with the film being solved and removed.

### Brief Description of the Drawings

Figure 1 is a schematic diagram illustrating an embodiment of a microcapsule production apparatus according to the present invention,
Figure 2 is a schematic diagram useful in understanding process for producing microcapsule according to the present invention, and
Figure 3 is a schematic diagram illustrating an example of an arrangement of microcapsules for a base material.

### Description of the Reference

1 base material
3 nozzle unit
4 drying unit

## Claims

1. A process for producing microcapsule comprising the steps of,
forming a first shell film by spotting a liquid droplet containing a first shell material to a base material (1),
forming a core layer by spotting a liquid droplet containing a core material to the first shell film, and
forming a second shell film by spotting a liquid droplet containing a second shell material to the base material so that the core layer is contained between the first shell film and the second shell film.

2. A microcapsule production apparatus comprising;
first shell film forming means (3)(4) for forming a first shell film by spotting a liquid droplet containing a first shell material to a base material (1),
core layer forming means (3) for forming a core layer by spotting a liquid droplet containing core material to the first shell film, and
second shell film forming means (3)(4) for forming a second shell film by spotting a liquid droplet containing a second shell material to the base material so that the core layer is contained between the first shell film and the second shell film.

3. A microcapsule sheet comprising;
a base material (1), and
microcapsules arranged at a predetermined pattern within a predetermined extent of the base material (1), the microcapsule being formed by surrounding a core layer with a first shell film and a second shell film.
